# EUROPEAN PATENT APPLICATION

(11) **EP 1 574 176 A1**
(43) Date of publication of application: **14.09.2005**
(21) Application number: 05251484.1
(22) Date of filing: 11.03.2005
(51) Int. Cl.: A61B 18/22

(54) **Medical apparatus and method for positioning an energy delivery device**

(30) Priority: 12.03.2004 US 799130
(71) Applicant: ETHICON ENDO-SURGERY, INC., Cincinnati, Ohio 45242 (US)
(72) Inventor: Speeg, Trevor, Williamsburg Ohio 45175 (US); Ritchie, Paul G., Loveland Ohio 45140 (US); Trusty, Robert M., Cincinnati Ohio 45241 (US); Nield, Scott A., Cincinnati Ohio 45215 (US)
(74) Representative: Tunstall, Christopher Stephen

(57) **Abstract**

A medical apparatus and method useful for the efficacious thermal treatment of lumen such as varicose veins during laser surgery is provided. An energy delivery device comprising a diffusing optical fiber with a light-emitting section and a memory device having data programmed therein is also provided. The optical fiber includes a temperature sensor for measuring a temperature at a treatment site. An energy generator is connected to the optical fiber and to a positioning device. The optical fiber engages positioning device so that the positioning device can moveably position the light-emitting section of the optical fiber. Consequently, the optical fiber can be inserted directly into an appropriate position within a varicose portion of a vein or other lumen for thermal treatment of the vein. The memory device and a main processor are used to automatically control the operation of the medical apparatus including the intensity of energy emitted and the movement or position of the light-emitting section of the optical fiber within the vein being treated.

## Description

### Field of the Invention

The present invention is related generally to a system for applying energy to human tissue, and more particularly, to such a system for treating a lumen such as a vein. The present invention also relates to a medical apparatus with an energy delivery device, and methods for use thereof, having capabilities to measure the temperature at the treatment site and control the amount of energy delivered while treating sections of the lumen.

### Background of the Invention

Currently physicians employ endovascular techniques to deliver laser energy conductively while treating blood vessels such as varicose veins. One such technique is described in U.S. Patent No. 6,398,777 issued to Navarro et al. on June 4, 2002 which discloses a method for treating varicose veins using a tipped laser energy carrier to deliver laser energy into the blood vessel lumen to produce vein wall damage with subsequent fibrosis. The method includes compressing the greater saphenous vein over the tip of the fiber optic line so that the tip of the fiber optic line makes direct contact with the vein wall while bursts of laser energy are delivered, and incrementally retracting the fiber optic line along the saphenous vein.

During the treatment of varicose veins using conductive energy delivery devices, accurately controlling the temperature achieved by the human tissue is desirable to assure efficacious treatment. One difficulty with the use of such typical fiberoptic technology to deliver energy to the wall of a vein or lumen is that unless the fiberoptic can evenly distribute energy radially outwardly, in a substantially uniform manner to the wall of the lumen, the shrinkage and fibrosis of the vein will not be uniform. An uneven distribution of energy will lead to detrimental results. Use of a diffusing fiberoptic can help avoid any uneven distribution of energy.

Physicians have used diffusing type energy delivery devices to treat damaged intervertebral disc when the damage has resulted in a bulge and where heating of the annulus will shrink the collagen in the annulus to help reduce the bulge. U.S. Patent No. 6,503,269 issued to Nield et al. on January 7, 2003 discloses a method for treating intervertebral discs including insertion of a light source into the damaged disc, activation of the light source to emit diffuse light, optically measuring the temperature of tissue near the light source, and modifying the intensity of the light emitted according to the measured temperature. While some fiber optic devices may be useful in controllably heating the annulus of an intervertebral disc, the anatomical differences and desired medical outcomes are distinctly different in the treatment of intervertebral disc and the incremental treatment along the length of a lumen such as a varicose vein. In the treatment of a varicose vein, the amount of energy absorbed by the vein can be monitored by measuring the temperature along each incremental segment of the vein while an entire length of the vein is being treated. Even minor variations in treatment effectively from one segment to another segment can change the therapeutic effects of the overall treatment.

Detrimental results may also occur if there is an over exposure at one treatment segment or under exposure at another treatment segment of the vein during the medical procedure as the fiber optic line is incrementally moved within the vein. Accurate measurement of the tissue temperature at each treatment segment can be used to assure the proper level or intensity of treatment is given along the length of the entire treatment site. In particular, any inconsistencies or shifts in the tissue temperature at a specific segment of the vein or along the length of the vein during treatment may indicate unwanted variations in energy delivery that may lead to over treatment or under treatment of the tissue, which can result in inferior clinical outcomes including failure to achieve fibrosis of the vein and additional surgical procedures.

Use of diffusion instead of conduction for energy delivery will assure a more even distribution of energy along the length of the vein. It is therefore desirable to utilize a diffusing fiberoptic device having the capability to monitor and control the temperature at each treatment segment and along the entire length of the vein by automatically controlling the amount of energy delivered at each treatment segment.

Consequently, there is a need for specific medical apparatuses that can assure an even distribution of energy along the entire length of the cylindrical surface of a lumen such as the saphenous vein. There is also a need for such devices that provide for monitoring of temperatures at each treatment segment while also providing for incrementally treating each segment along the length of the lumen. Such an apparatus and methodology will help assure that patients receive the most efficacious treatment that their physicians can provide.

### Summary of the Invention

The present invention provides for the use of a medical apparatus to assist in the efficacious treatment of patients during laser surgery. In one embodiment, the present invention provides a medical apparatus for the thermal treatment of human tissue that includes an energy delivery device. The energy delivery device includes an optical fiber having a diffusing, light-emitting section at a distal end. A positioning device engages the optical fiber. The positioning device moves and positions the light-emitting section within the human tissue. The human tissue can be a lumen in the form of a blood vessel or vein. An energy generator is included that has a main processor for controlling the operation of the medical apparatus. The positioning device is operatively connected to the energy generator and the main processor controls the positioning of the light-emitting section using the positioning device. A single treatment site can be divided into a multiplicity of treatment segments and the light-emitting section of the optical fiber is moved from one treatment segment to another. The light-emitting section of the optical fiber can be moved in either an incremental or a continuous manner. The light-emitting section can be aligned with a treatment segment visually using light emitted from a visible wavelength marker laser. The optical fiber can include a temperature sensor for optically measuring a temperature within the human tissue at the treatment segment. The energy delivery device can also include a memory device having parameters stored therein. The main processor can compare the optically measured temperature to at least one of the parameters stored in the memory device. The main processor can automatically control the positioning of the light-emitting section within the treatment site and can also automatically adjust the energy delivered to the light-emitting section from the energy generator in response to the measured temperature.

Additional advantages and features of the present invention will become more apparent from the following detailed description which may be best understood with reference to and in conjunction with the accompanying drawings.

### Brief Description of the Figures

FIGURE 1 is an isometric view of a medical apparatus, including an energy generator, an energy delivery device and a positioning device according to one embodiment of the present invention;

FIGURE 2 is an isometric view of the energy generator of Figure 1 with the cover removed for clarity;

FIGURE 3 is an isometric view of the connector of Figure 1;

FIGURE 4 is a sectional view taken in side elevation along the centerline of the connector shown in Figure 3;

FIGURE 5 is a plan view showing an opposite side of the printed circuit board of Figure 4;

FIGURE 6 is a sectional view taken in side elevation of the optical fiber of Figure 1;

FIGURE 7 is a plan view of one embodiment of the positioning device of Figure 1 with the panel removed for clarity;

FIGURE 8 is a schematic illustrating a method for use of the medical apparatus in accordance with the present invention;

FIGURE 9 is a cross sectional schematic illustrating use of the optical fiber in accordance with the present invention; and

FIGURE 10 is a block diagram illustrating a method for use of the medical apparatus in accordance with the present invention.

### Detailed Description of the Invention

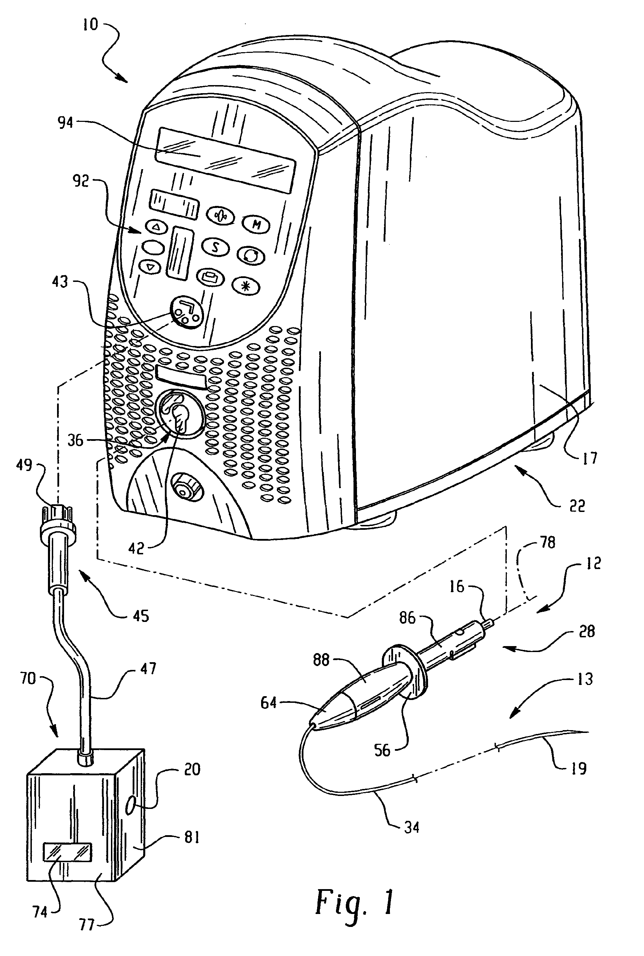

In this detailed description of the present invention, any patent or non-patent literature referenced herein and the disclosure contained therein is intended to be and is hereby incorporated by reference. Additionally, in this description of preferred embodiments, "means for generating energy" and "energy generator," "energy source," "generator" or "generating means" or the like, can be used interchangeably and, similarly, "delivering means" and "energy delivery device," "delivery device" or the like, can be used interchangeably unless otherwise specified. Additional terms may be used in the same manner, as will be clear to the reader. Further, the terms "proximal" and "distal" are used to refer to relative locations nearest to and farthest from, respectively, the ferrule 16 in connector 28 of the energy delivery device 12 of the medical apparatus 10, as shown in Figure 1. These conventions are adopted merely by way of convenience, not by way of limitation.

Referring now to the Figures in which like numerals indicate like elements, Figure 1 discloses medical apparatus 10 useful for transferring diffused light energy to human tissue which includes energy generator 22, positioning device 70 and energy delivery device 12, illustrated in a disconnected configuration. In the preferred embodiment of an energy generator 22 shown, energy is generated in the form of laser light. Nonetheless, energy generator 22 could be any means for generating energy or a generator for many different types of energy such as, for example, laser light energy, infrared energy, radio frequency energy, microwave energy, ultrasound energy or any other energy suitable for the treatment of human tissue. By way of example, a means for generating ultrasonic energy may be the Ultracision Harmonic Scalpel commercially available from Ethicon Endo-Surgery Inc., of Cincinnati, Ohio, and a means for generating radio-frequency energy may be any of a variety of surgical generators, such as the ICC 350 Electrosurgical Generator commercially available from Erbe USA, Inc., of Marietta, Georgia. Preferably, energy generator 22 is a portable diode based laser, and most preferably, the Indigo® Optima laser system commercially available from Ethicon Endo-Surgery, Inc. of Cincinnati, Ohio.

A cover 17 shields interior components of energy generator 22. A connector housing 36 and a receptacle 43 reside within a front portion of cover 17. Both the front of connector housing 36 and the front of receptacle 43 are exposed to the exterior. Medical apparatus 10 further includes an energy delivery device 12 having connector 28 at its proximal end and optical fiber 13 at its distal end. The optical fiber 13 of energy delivery device 12 extends from connector 28 to light-emitting section 19. Optical fiber 13 could be associated with any energy delivery device 12 capable of delivering useful energy such as, for example, laser light energy, infrared energy, radio frequency energy, microwave energy, ultrasound energy or any other energy suitable for the treatment of human tissue. Energy delivery device 12 could be any means for delivering energy or any device capable of delivering many types of useful energy from the energy generator 22.

Energy delivery device 12 is attachable to connector housing 36 by inserting connector 28 through an opening 42 in connector housing 36 to lock the connector 28 in position. Connector 28 inserts into connector housing 36 and locks into connector housing 36 by rotation about a longitudinal axis 78. In one embodiment, energy delivery device 12 may be a disposable delivery device with a limited useful life, including data stored therein in the form of use parameters and properties, for delivering energy from an energy generator 22 to human tissue. In this embodiment, energy delivery device 12 can be removed from energy generator 22 by unlocking connector 28 from connector housing 36 by rotation about a longitudinal axis 78 in a direction opposite the locking rotation.

Positioning device 70 is operatively connected to energy generator 22 through wire harness 47. Wire harness 47 is attachable to receptacle 43 by inserting prongs 49 of plug 45 into receptacle 43. Positioning device 70 transmits and receives energy and data from energy generator 22 which energizes the positioning device 70. A panel 77 having remote or touch screen 74 included thereon covers the internal contents of positioning device 70 contained within housing 81. Wire harness 47 has plug 45 at one end thereof and is connected to the internal contents of housing 81 at the other end. Optical fiber 13 can engage positioning device 70 through aperture 20 in housing 81. Positioning device 70 is used to continuously or incrementally move optical fiber 13 of energy delivery device 12. Increment or incrementally as used herein means non-continuous.

As shown in Figure 1, the energy generator 22 may include a keypad 92 on cover 17 for user interface and input of data. The energy generator 22 may also include a display screen 94 on cover 17 for the display of data, warnings, or other information. The positioning device 70 may also include a remote or touch screen 74 on panel 77 for the display of data, warnings, or other information remotely from energy generator 22. Thus, the physician need not look away from the general area of the patient being treated to read such information.

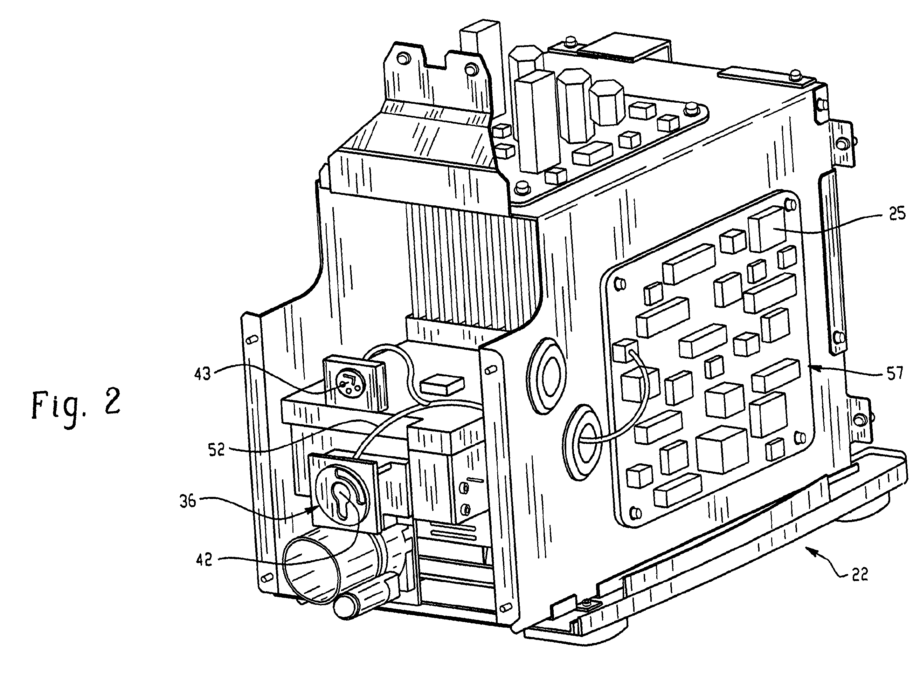

Figure 2 depicts energy generator 22 with cover 17 removed to expose interior portions of energy generator 22. In this embodiment, conductor cable 52 electrically joins connector housing 36 and receptacle 43 to controller board 57 on energy generator 22. Located on controller board 57 is a computer in the form of main processor 25, which receives and processes electronic signals to control the operation of medical apparatus 10. Main processor 25 can be, for example, a microprocessor or miniature computer. Signals from electronic components within energy delivery device 12 and positioning device 70 communicate via conductor cable 52 with controller board 57 and main processor 25. Alternatively, separate conductor cables 52, controller boards 57, and main processors 25 could be used for each component. Additionally, the main processor 25 can be operatively connected to the keypad 92 and the display screen 94.

In operation, the main processor 25 directs the energy application process according to instructions from the user via the keypad 92 or programmed instructions and data from the energy delivery device 12 and positioning device 70, as further described herein. The main processor 25 communicates information concerning the process to the display screen 94 and/or remote screen 74 for observation by the user. Main processor 25 may also enunciate information in an audible manner using methods known in the art. Should the user find the information concerning the process undesirable, for example, unsafe to the patient undergoing treatment, he or she may override the operating instructions via the keypad 92.

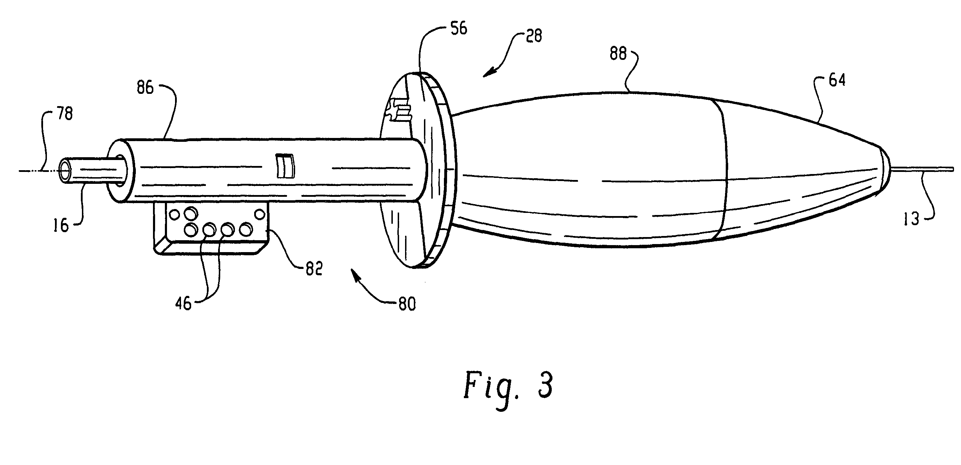

As shown in Figure 3, connector 28 possesses a handle portion 88, shaped for easy grasping by the user, and capped on the distal end with a boot 64. Optical fiber 13 extends distally from the boot 64. A barrel 86 continues proximately from handle portion 88. A connector face 56 separates barrel 86 from handle portion 88. Attached to barrel 86 is a flange 82 radially extending from longitudinal axis 78. Flange 82 includes contact pad access openings 46 placed on a large side of flange 82. An axial gap 80 separates the distal end of flange 82 from connector face 56. Ferrule 16 is located within connector 28 and a portion of ferrule 16 protrudes from the proximate end of barrel 86. Ferrule 16 is one form of an energy transfer attachment for transferring energy from energy generator 22 to energy delivery device 12 for medical treatment. Opening 42 on connector housing 36 allows entrance of barrel 86 of connector 28 to operatively connect the energy delivery device 12 to the energy generator 22.

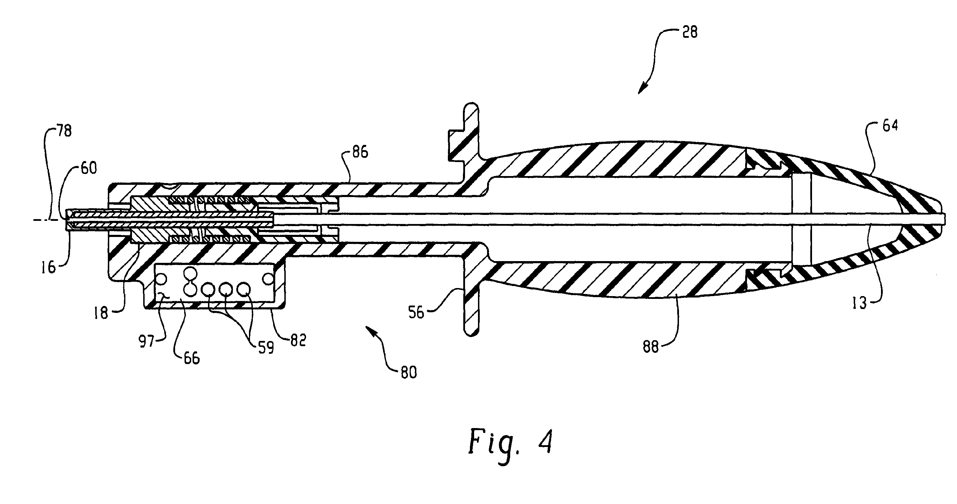

A cross sectional view of connector 28 is shown in Figure 4 depicting the interior portions of connector 28. Ferrule 16 has a passageway 60 through the center thereof to admit light energy generated by energy generator 22 into optical fiber 13. The passageway 60 in ferrule 16 is coaxial with longitudinal axis 78. The interior of handle portion 88 engages enlarged portion 18 of ferrule 16 and boot 64 surrounds and retains optical fiber 13 as it emerges from handle portion 88 of connector 28. Printed circuit board 66 within flange 82 is also illustrated with mating surface 97. Printed circuit board 66 can be inset-molded into flange 82 leaving only contact pads 59 open to the exterior through access openings 46. Connector 28 is preferably molded of non-conductive material such as plastic.

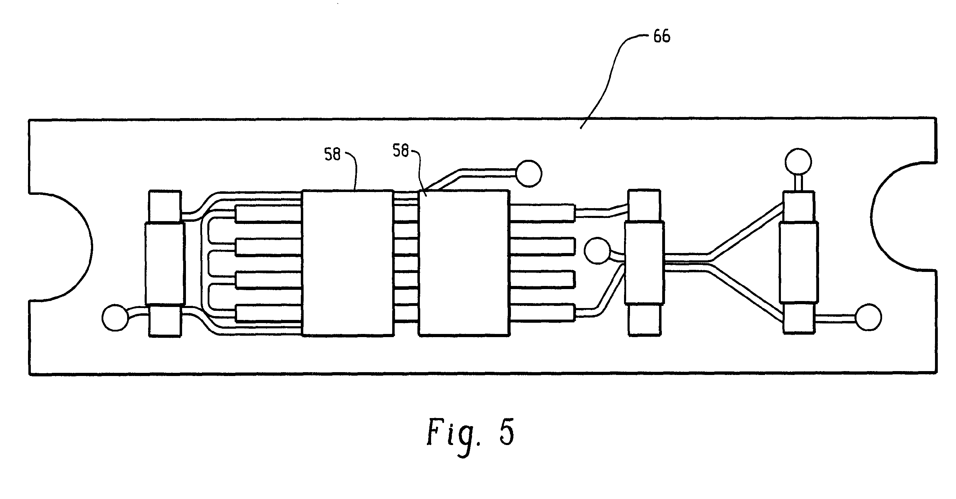

Figure 5 depicts the side of printed circuit board 66 opposite that shown in Figure 4. At least one memory device 58 resides on the side of printed circuit board 66 opposite mating surface 97 and is in electrical communication with contact pads 59. Memory device 58 can be, for example, an electronic erasable programmable read-only memory device (EEPROM) and can store information useful to the operation of energy delivery device 12 and medical apparatus 10.

With connector 28 in the locked position, memory device 58 can communicate electrically with main processor 25 on controller board 57 through contact pads 59 and conductor cable 52. Information within memory device 58 may now be accessed by main processor 25 and vice versa.

While the memory device 58 has been described as an EEPROM, which may store a significant amount of data, it may alternatively be any non-volatile type memory of a variety of digital, optical, or magnetic memory storage devices or integrated circuits providing memory capability. For example, such memory device 58 may include read-only memory (ROM), programmable read-only memory (PROM), erasable programmable read-only memory (EPROM), flash memory, non-volatile random access memory (RAM), or most preferably EEPROM. Of course, the entire set of data or information need not be stored in a single memory device 58 or in a single type of memory device 58, for it is understood that multiple memory devices 58 of multiple types can be used in accordance with the present invention. Further, while the memory device 58 has been described as being mounted on printed circuit board 66 which is inset molded on flange 82, it is understood that printed circuit board 66 or memory device 58 can alternatively be externally mounted or even a wholly separate assembly or device that operatively connects to energy generator 22 or energy delivery device 12 via a separate electrical connection or some other method of connection. Additionally, memory device 58 can be operatively connected to optical fiber 13 from a location remote from energy delivery device 12 without varying from the scope of this invention. Operatively connected as used herein refers to the ability of components to transmit energy or to exchange data such as via the communication of electronic data between each component. Moreover, while the exchange of data between the memory device 58 and the energy generator 22 has been described as possibly being accomplished via electrical means, it may alternatively be accomplished via magnetic, infrared, radio frequency or even optical means. These alternatives and others, which may be arrived at by one of ordinary skill in the art without undue experimentation, and are contemplated as being within the scope of the present invention.

An energy delivery device 12 used for these purposes typically extends from a connector 28 to at least the distal end of the optical fiber 13. Preferably, the energy delivery device 12 includes a means for emitting energy from the energy delivery device 12 to the human tissue at or near its distal end. In particular, medical apparatus 10, with energy delivery device 12, can be used to apply laser light energy to human tissue for therapeutic treatment of the human tissue, for example, the thermal treatment of blood vessels and veins or other lumen. The term "lumen" as used herein refers to the bore or cavity of a tubular organ.

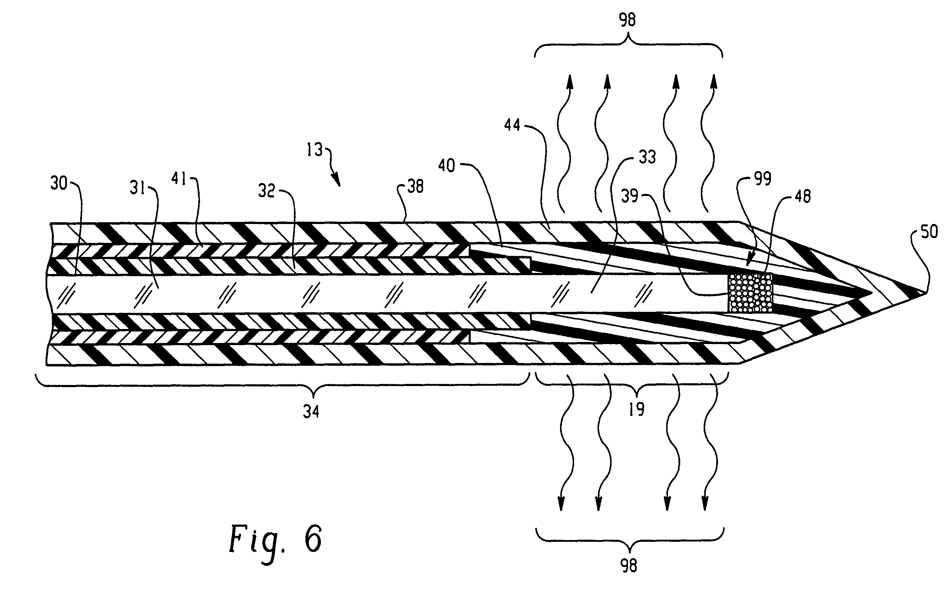

Now referring to Figure 6, an energy delivery device 12 according to one embodiment of the present invention, includes an optical fiber 13 comprising a diffuser or light-emitting section 19 at its distal end and a non-diffusing or light-transmitting portion 34 extending toward its proximal end. In light-transmitting portion 34 of optical fiber 13, a cladding 32 and the proximal portion of a sheath or sleeve 38 radially surround the proximal portion 30 of core 31. Optical fiber 13 may have a jacket or buffer layer 41 arranged to extend circumferentially between the cladding 32 and the sleeve 38. The material used to form the cladding 32 has an index of refraction lower than the index of refraction of the material used to create the glass or core 31 so as to contain the light within the core 31 throughout the length of the light-transmitting portion 34. In light-emitting section 19 of optical fiber 13, the core 31 extends beyond its proximal portion 30 through a distal portion 33 to the distal end 39 thereof. The distal portion 33 of the core 31, which is employed to diffuse light, is surrounded by an optical coupling layer 40 and the distal portion 44 of the sleeve 38 thereby forming the light-emitting section 19 without the cladding 32 of the light-transmitting portion 34. Arrows 98 illustrate the diffuse light energy being emitted and radiated outwardly from light-emitting section 19 evenly in all radial directions.

A material having an index of refraction higher than the index of refraction of the core 31 forms the optical coupling layer 40. Preferably, UV50 Adhesive, commercially available from Chemence, Incorporated, in Alpharetta, Ga., is the adhesive used to produce the optical coupling layer 40. Other adhesives which may be used include XE5844 Silicone, available from General Electric Company and 144-M available from Dymax of Torrington, CT.

The sleeve 38 can extend distally past the distal end 39 of the core 31 and may be configured to form tip 50. In one embodiment, tip 50 is formed to a sharp or pointed tip 50 capable of piercing through human tissue in order to enable some medical procedures. In another embodiment, tip 50 may take on other forms and configurations, for example, a rounded, bulbus or blunt tip 50 can be used in the treatment of varicose veins where optical fiber 13 is introduced into the human tissue through a cannula. In a preferred embodiment, sleeve 38 of optical fiber 13 constitutes one continuous piece and, more preferably, sleeve 38 comprises perfluoroalkoxy impregnated with barium sulfate.

A light-scattering component 48, which is filled with a light-scattering material, is located on the distal end 39 of core 31 and can reflect light back into the core 31 so as to provide a more even or uniform light distribution. In a preferred embodiment, alexandrite particles are employed as the light-scattering material for light-scattering component 48. In addition to its light-scattering properties, the light-scattering component 48 fluoresces in a temperature-dependent manner upon being stimulated by light. For example, some of the light energy absorbed by the light-scattering component 48 causes the stimulation of the light-scattering component 48 which then generates and releases light energy back into the core 31 toward the proximal end in the form of a temperature signal having a longer wavelength and a phase or time delay. The frequency or time delay between the light energy absorbed by the light-scattering component 48 and the emission of the light energy from the light-scattering component 48 is dependent on the temperature of light-scattering component 48. Main processor 25 calculates a temperature by use of this phase difference or temperature signal, which it converts into a temperature measurement. It is this temperature-dependent fluorescence property of the light-scattering component 48 that is adapted to be used as a temperature sensor 99. Thus, the fluorescent properties of the alexandrite particles, when stimulated by light energy of the proper wavelength, can allow the determination of the temperature of human tissue surrounding light-emitting section 19 by methods which are known in the art. In this closed loop manner, an indication or measurement of temperature in the human tissue at the treatment location in proximity to the light-emitting section 19 or tip 50 is measured optically.

A variety of data and information can be converted into digital form and then loaded, stored or programmed into memory device 58. Methods of storing this data and information in a digital form are well known in the art. Parameters are used or established that relate to this particular data and information. The word "parameter," as used herein, is used as a symbol representing variables, functions, constants, and parametric equations.

By way of example, usage-related parameters can be preset during manufacture or can be set during use and may include or be derived from data and information relating to the medical apparatus 10 that is static (having a fixed value) or that is dynamic (having a changeable or variable value) such as any of the following:
identification of the delivering means; expiration, or non-expiration date of the delivering means; calibration parameters; scale and offset factors; self heating characteristics; type of energy delivery; operational parameters; energy delivery parameters; monitoring sequence parameters; identification of the generating
means; amount of energy delivery; maximum power; power range; power transmittance; wavelength; data integrity factors; time from the initial recognition of the energy source; identification numbers; lot numbers; expiration date; prior usage history; energy delivery time; rate of energy delivery; rate of insertion or retraction; total joules delivered; number of treatment sites; dimensional characteristics of treatment site such as length, diameter, thickness, etc.; identification, type, date, or time of treatment; total treatment time; duration of treatment; time of treatment at each segment; treatment type; characteristics of human tissue to be treated; mode of operation; elapsed time; total elapsed time of all treatments; temperature levels at treatment segments; target temperature; maximum temperature; identification of multiple generating means; historical data regarding attainment of certain temperature levels or power levels; historical data regarding use by multiple generating means; indication or identification of error or warning; or any abnormal or premature termination of treatment including any problem conditions triggered during any treatments; and any combination or combinations thereof. Such usage-related parameters may also include various other data and information relating to the operation of optical fiber 13, energy delivery device 12, positioning device 70, energy generator 22, or medical apparatus 10.

Main processor 25 may use the data and information stored within memory device 58 to automatically modify the intensity or energy output of energy generator 22. Also, main processor 25 may make decisions regarding the information contained within memory device 58. For example, when power is applied to activate or energize positioning device 70 or energy delivery device 12, main processor 25 may increase, decrease, disable, or even shut off the energy delivered by energy generator 22 based on the particular data and information communicated between the main processor 25, memory device 58 and positioning device 70. As a further example, main processor 25 may generate messages including error messages regarding the data and enunciate them audibly or display them on display screen 94 of energy generator 22 or remote screen 74 or positioning device 70. Main processor 25 may even write information to memory device 58 to be stored in memory device 58 with energy delivery device 12.

Preferably, energy delivery device 12 with connector 28 is the fiberoptic system associated with the Indigo® Optima laser system, which is commercially available from Ethicon Endo-Surgery Inc., Cincinnati, Ohio. Energy delivery device 12 along with energy generator 22 are further described and disclosed in commonly assigned U.S. Patent No. 6,503,269, entitled "Method Of Treating Intervertebral Discs Using Optical Energy And Optical Temperature Feedback" issued to Nield et al. on January 7, 2003; U.S. Patent No. 6,522,806, entitled "Optical Fiber Including A Diffuser Portion And Continuous Sleeve For The Transmission Of Light" issued to James, IV et al. on February 18, 2003; U.S. Patent Application Publication No. 2002/0186748, entitled "System And Method Of Measuring And Controlling Temperature Of Optical Fiber Tip In A Laser System" by Yates et al. and published on December 12, 2002; U.S. Patent Application Publication No. 2001/0025173, entitled "Energy Application System With Ancillary Information Exchange Capability, Energy Applicator, And Methods Associated Therewith" by Ritchie et al. and published on September 27, 2001; U.S. Patent Application Publication No. 2002/0081871, entitled "Connector Incorporating A Contact Pad Surface On A Plane Parallel To A Longitudinal Axis" by Swayze et al. and published on June 27, 2002; U.S. Patent Application Publication No. 2003/0118302, entitled "Optical Fiber Including A Diffuser Portion And Continuous Sleeve For The Transmission Of Light" by James, IV et al. and published on June 26, 2003; U.S. Patent Application Serial No. 10/721,111, entitled "Energy Delivery Device With Self-Heat Calibration" filed on November 25, 2003; and U.S. Patent Application Serial No. 10/723,799, entitled "Method Of Limiting Re-use For Energy Deliverables" filed on November 26, 2003.

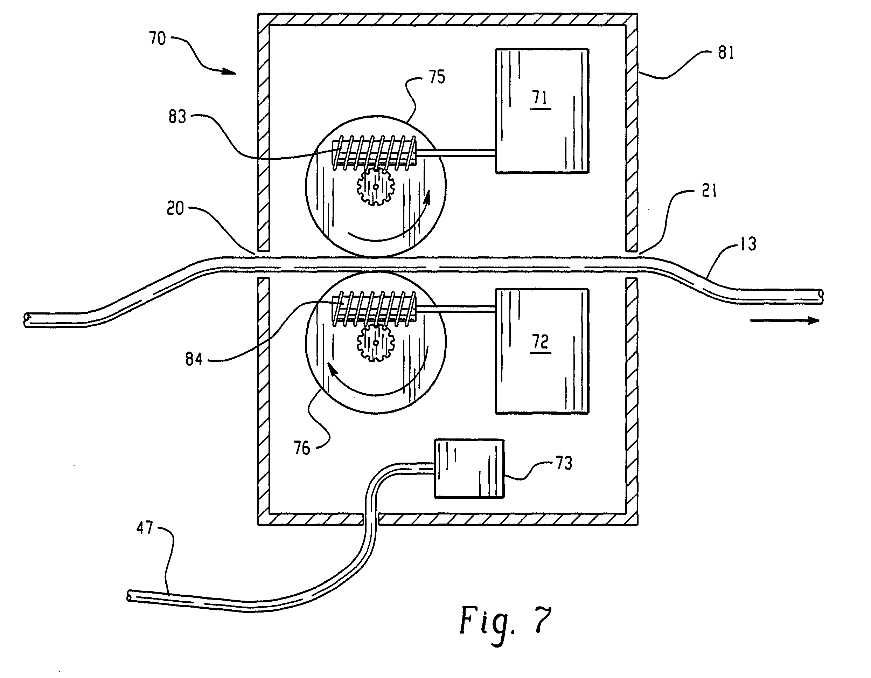

Figure 7 depicts positioning device 70 with panel 77 removed to expose interior portions of positioning device 70. In the embodiment shown, wire harness 47 electrically connects remote processor 73 to energy generator 22 and the other components of medical apparatus 10. First motor 71 and second motor 72 are operatively connected to remote processor 73. Remote processor 73 can therefore receive and process electrical signals from main processor 25 to control the operation of first motor 71 and second motor 72 of positioning device 70. First motor 71 can be directly linked to first roller 75 through first drive 83. Similarly, second motor 72 can be directly linked to second roller 76 through second drive 84. First roller 75 and second roller 76 are spaced apart but located immediately adjacent to each other and are preferably made of a rubber material. During operation first motor 71 and second motor 72 convert electrical energy into mechanical energy causing the first drive and second drive respectively to rotate first roller 75 and second roller 76 respectively in rotationally opposite directions. The spacing between first roller 75 and second roller 76 allows optical fiber 13 to pass between the rollers while still contacting each individual roller as it passes there between. In other words, first roller 75 and second roller 76 engage optical fiber 13 and govern the movement of optical fiber 13 as it is squeezed between first roller 75 and second roller 76. As illustrated, optical fiber 13 is engaged by first roller 75 having a substantially counterclockwise rotation and is also engaged by second roller 76 having a substantially clockwise rotation which allows optical fiber 13 to be moved in the distal direction. When the rotation of first roller 75 and second roller 76 is reversed, optical fiber 13 will move in the proximal direction. First motor 71 and second motor 72 are synchronized in a manner that assures first roller 75 rotates in a direction opposite second roller 76. Preferably, the rotation of first roller 75 and second roller 76 is smooth over their entire range of motion. More preferably, the rate of rotation of first roller 75 and second roller 76 can be varied over a wide range of speeds and can even be stopped for an increment of time at any point during the rotation. In this manner positioning device 70 moves the light-emitting section 19 of optical fiber 13 in either a distal or proximal direction and light-emitting section 19 can be moved in either a continuous or incremental manner.

Upon connection of the energy delivery device 12 and positioning device 70 to each other and to energy generator 22, the energy delivery device 12 is ready to receive energy from the energy generator 22 and deliver the energy to the human tissue from its light-emitting section 19 of optical fiber 13 in accordance with the present invention.

In one alternative embodiment, the positioning device 70 can have a wiring harness 47 that is directly connected to an ordinary electrical outlet. In this embodiment, movement of light-emitting section 19 of optical fiber 13 within vein 91 can be directed by the physician manually entering commands into positioning device 70 using remote or touch screen 74 or by using other methods of inputting commands to operate positioning device 70 as is well known in the art. In response to such commands, remote processor 73 can control the rotation of first roller 75 and second roller 76 to properly position light-emitting section 19 as previously described.

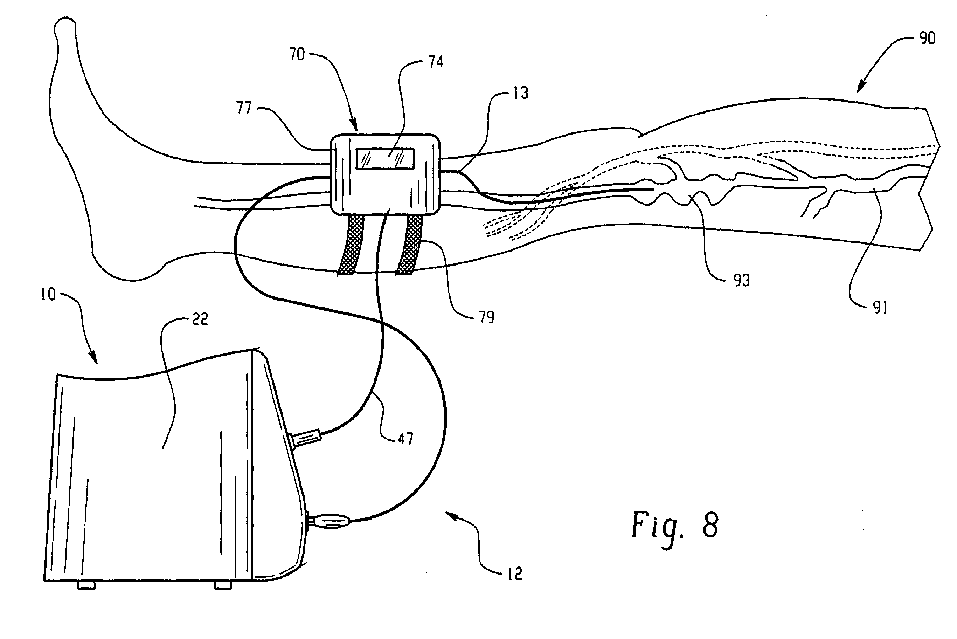

Referring now to Figure 8, energy generator 22 is connected to energy delivery device 12 and positioning device 70. Energy delivery device 12 includes optical fiber 13 having a light-emitting section 19 and a temperature sensor 99 at a distal end for generating a temperature signal in the previously identified closed loop manner. Optical fiber 13 engages first roller 75 and second roller 76 of positioning device 70 as optical fiber 13 passes into housing 81 through aperture 20 and out of housing 81 through aperture 21. Optical fiber 13 can be inserted directly into vein 91 to an appropriate position in a varicose portion 93 of vein 91 within the human tissue or leg 90 of the patient as determined by the physician. Preferably, positioning device 70 is attached to the leg 90 of a patient using support straps 79. Alternatively, positioning device 70 can be freestanding immediately adjacent to the patient and leg 90 utilizing a support frame in lieu of support straps 79 to hold positioning device 70.

In this embodiment of the present invention, the medical apparatus 10 includes energy generator 22, positioning device 70 and energy delivery device 12. Wire harness 47 operatively connects energy generator 22 and positioning device 70. Main processor 25 within energy generator 22 is used to control the operation of medical apparatus 10 including the positioning and repositioning of light-emitting section 19 of optical fiber 13 within vein 91 of leg 90 by positioning device 70. In this configuration, medical apparatus 10 is ready to emit laser light energy for the treatment of vein 91.

During normal operation of medical apparatus 10, different wavelengths of light energy are generated by energy generator 22 in the form of a treatment laser and a marker laser. This light energy travels through core 31 to light-emitting section 19. At light-emitting section 19 the light energy is emitted from core 31 through optical coupling layer 40 since optical coupling layer 40 has a higher index of refraction than core 31. The distal portion 44 of sleeve 38, which surrounds optical coupling layer 40, preferably uses barium sulfate particles scattered within sleeve 38 to diffuse the light energy radially outwards towards the human tissue. Light-emitting section 19 is used to scatter and diffuse this light energy into the treatment site thereby heating the treatment site. The treatment laser is light energy having a wavelength of between about 810 nm to about 830 nm and is the energy used to thermally treat vein 91. The marker laser is light energy having a wavelength of about 635 nm, which is within the visible spectrum, and is used to stimulate temperature sensor 99. The pulsed light energy reaching light-scattering component 48 is absorbed and reemitted back towards core 31 at a wavelength that is different from the marker laser and delayed in phase from the marker laser by the alexandrite particles in light-scattering component 48. This marker laser light energy can also be used by the physician to identify the position of light-emitting section 19 since this light energy is visible through the human tissue. In particular, optical fiber 13 can have position markings visible on sleeve 38 of optical fiber 13. These position markings can be used by the physician to identify the position of light-emitting section 19 and the amount of optical fiber 13 inserted into the patient's leg 90.

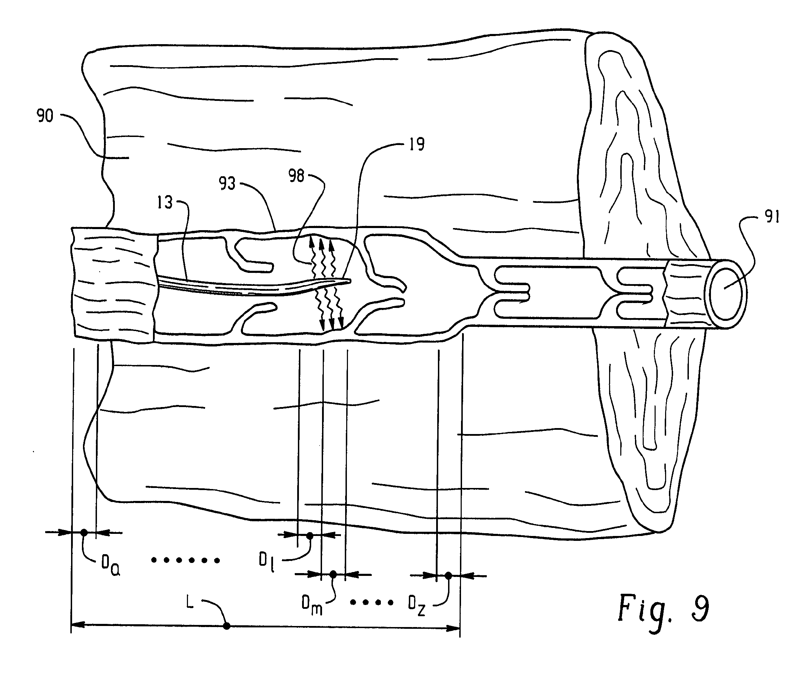

Figure 9 illustrates optical fiber 13 and its light-emitting section 19 positioned within a varicose portion 93 of vein 91. The multiplicity of adjacent treatment segments are indicated generally as lengths Dₐ through D_{z}. The treatment segment Dₘ is shown as being treated by the light energy, indicated generally by arrows 98, emitting radially outwardly from light-emitting section 19. The entire varicose portion 93 of vein 91 can be the treatment site illustrated as length L. Each treatment segment Dₐ through D_{z} is a portion of the overall treatment site L. In other words, treatment site L comprises treatment segments Dₐ + ... + Dₗ + Dₘ + ... + D_{z}. The multiplicity of treatment segments Dₐ through D_{z} can be incrementally treated with laser light energy 98. When the thermal treatment of treatment segment Dₘ is completed optical fiber 13 can be moved or repositioned in an incremental or a continuous manner to the next or adjacent treatment segment D₁ within or along the entire length of treatment site L by positioning device 70. Energy delivery device 12 can be energized in a corresponding incremental manner by energy generator 22 providing laser light energy 98 to treat each new treatment segment D₁. This process can be repeated until the entire treatment site L of vein 91 has been treated with laser light energy 98.

In a preferred embodiment, the data stored within memory device 58 can relate directly to the length, thermal energy, and treatment temperature for the particular treatment site L. Thus memory device can transmit information to main processor 25 which in turn will direct the movement of optical fiber 13 as well as the intensity of laser light energy that is emitted by light-emitting section 19 into each particular treatment segment D within treatment site L. When the expected or target temperature is detected at treatment segment D, main processor 25 can retract or advance optical fiber 13 using positioning device 70 to align light-emitting section 19 with the next or adjacent treatment segment D. Alternatively, the information and data programmed into memory device 58 can relate to the dimensional characteristics of varicose portion 93 of vein 91 such that the incremental retraction of optical fiber 13 and corresponding thermal treatments of vein 91 can occur automatically. In this manner, optical fiber 13 can be retracted at a constant or substantially continuous rate that enables the temperature at a particular treatment segment D to be achieved or can be retracted incrementally by treating each discrete treatment segment D prior to automatically being retracted to the next incremental treatment segment D along length L. Since the characteristics regarding varicose portion 93 of vein 91 and any particular characteristics of the patient's leg 90 can all be programmed into memory device 58 along with a multiplicity of other treatment parameters including preferred temperatures and rates of retraction, the medical apparatus 10 can be programmed to automatically treat the patient in an incremental manner while optical fiber 13 is being retracted or moved along varicose portion 93 of vein 91. Preferably, positioning device 70 moves or retracts light-emitting section 19 at a rate of movement that assures an appropriate distribution of light energy 98 within each treatment segment D. This movement of light-emitting section 19 can be on a schedule preset from data stored in memory device 58, and more preferably can be at a rate of movement from between about 2.54 cm/minute to about 25.4 cm/minute.

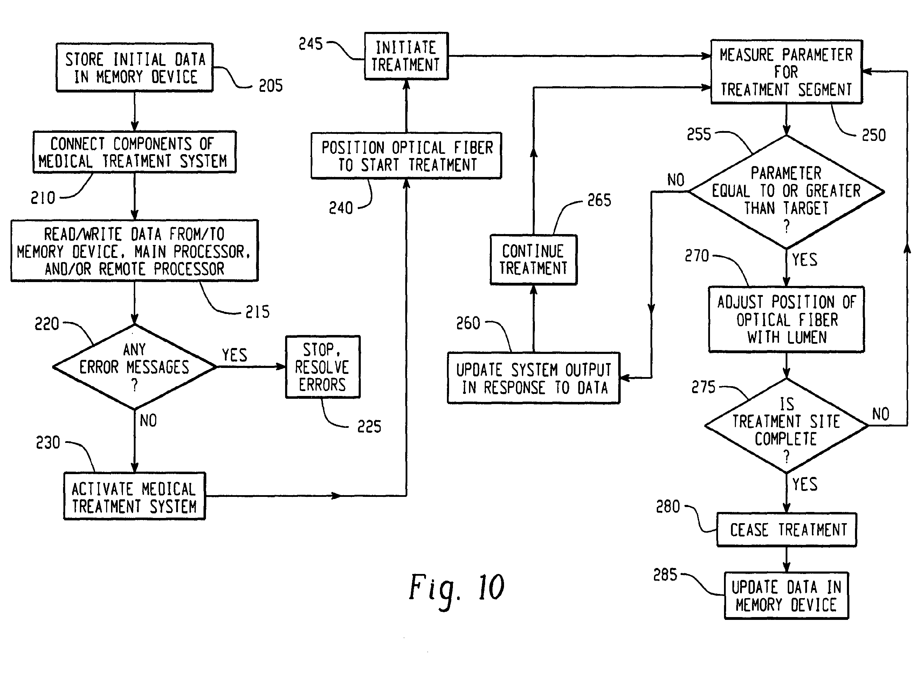

Now referring to Figure 10, it will be apparent to those of ordinary skill in the art that the previously identified data and information can be stored in memory device 58 in a variety of ways known to those of ordinary skill in the art [205]. In this embodiment of the invention, the preferred manner of operatively connecting energy delivery device 12 and positioning device 70 to energy generator 22 is by a direct electrical connection [210]. Upon engaging memory device 58 of energy delivery device 12, main processor 25 of energy generator 22 and remote processor 73 of positioning device 70 can read the data and information from, or write data and information to, memory device 58 [215]. Any programming or input of patient specific data from the physician can also occur. For example, the physician can program the medical apparatus 10 or memory device 58 by storing the length of treatment site L of vein 91 to be treated including any particular length of desired treatment segments D. The display screen 94 and remote screen 74 can be consulted for any error messages or other prompts [220]. In the event that error messages occur, the physician can stop and resolve any such errors or problems prior to proceeding with the treatment [225].

The physician can activate medical apparatus 10 to view the marker laser light after insertion into the human tissue [230]. The optical fiber 13 and light-emitting section 19 can be positioned in treatment site L at the appropriate treatment segment D [240]. Then treatment can be initiated. The physician will initiate treatment so that main processor 25 will prompt energy generator 22 to allow the appropriate intensity of energy to be emitted through light-emitting section 19 [245]. Upon activation, temperature sensor 99 can send back a temperature signal to main processor 25 corresponding to the measured temperature at treatment segment D [250]. Parameters other than temperature can be identified and measured if appropriate. The measured temperature is then compared to a temperature target stored in memory device 58 [255]. The target temperature may vary from treatment segment Dₐ to treatment segment D_{z} within treatment site L. If the temperature target is not yet achieved, main processor 25 can increase the energy output through light-emitting section 19 or can adjust the rate of movement of light-emitting section 19 in response to the measured temperature [260]. The treatment can continue in this manner until the particular parameter measured equals the target parameter [265].

Main processor 25 can adjust the position of or reposition optical fiber 13 so that light-emitting section 19 moves to the next treatment segment D within the lumen [270]. In particular, main processor 25 can communicate with remote processor 73 to activate positioning device 70 engaging first motor 71 and second motor 72 to move fiber optic 13 a specific distance based on the length of treatment segment D within vein 91 that the physician initially programmed into medical apparatus 10 or that was stored in memory device 58. Medical apparatus 10 can perform a treatment by emitting light energy at an intensity determined by main processor 25 into that particular treatment segment D and thereafter, the process can be repeated automatically until the entire length of treatment site L of vein 91 has been treated [275].

Alternatively, the physician can program memory device 58 and medical apparatus 10 for the entire length of treatment site L of vein 91 and set a temperature target and thereafter initiate the treatment as previously described. Energy generator 22 will transmit light energy through fiber optic 13 and emit light energy 98 through light-emitting section 19 into the varicose portion 93 of vein 91 until the temperature measured by temperature sensor 99 at the treatment segment D reaches the predetermined temperature target. The temperature at the treatment segment D is determined utilizing temperature sensor 99 in the close loop manner previously described. Main processor 25 can activate positioning device 70 to continuously retract or move optical fiber 13 through vein 91. The rate at which optical fiber 13 is withdrawn and/or the energy level and the power intensity are controlled by main processor 25 based on data stored in memory device 58 in order to maintain the desired target temperature at each treatment segment D. The rate of withdrawal as well as intensity of energy emitted can be automatically and continuously adjusted throughout the entire length of treatment site L of the vein 91. In this manner medical apparatus 10 assures the most effective treatment of vein 91 throughout the entire treatment cycle. Alternatively, the retraction of optical fiber 13 from vein 91 can be in small incremental steps or locations, as indicated by treatment segment D, and the movement between incremental steps can be at a continuous rate or a variable rate. Light-emitting section 19 can even have a predetermined dwell time between each incremental step.

In an alternative embodiment of the present invention, medical apparatus 10 can be operated without positioning device 70. The physician can simply manually insert and move and retract optical fiber 13 from vein 91 using the marker laser light and position markings to properly position light-emitting section 19. The physician can grip optical fiber 13 and push or pull on optical fiber 13 to position or align light-emitting section 19 within varicose portion 93 of vein 91. Upon initiation of treatment, the physician can manually retract optical fiber 13 using the temperature measurements from temperature sensor 99 displayed on remote screen 74 or display screen 94 as visual cues regarding the rate at which the physician should move light-emitting section 19 from one treatment segment D to another within treatment site L and along a length of vein 91.

After applying energy to the human tissue and completion of the medical procedure, the treatment can be ceased [280]. Data relating to the medical procedure or any information useful for medical apparatus 10 can be updated in memory device 58 [285]. The optical fiber 13 can be removed from the lumen and the medical apparatus 10 shut down. The user can remove plug 45 from receptacle 43 and connector 28 from connector housing 36 for convenient storage of these components. While plug 45 can be removed by just pulling it away from receptacle 43, to remove connector 28 the user needs to rotate connector 28 from the locked position to an unlocked position. After rotating connector 28, the user can pull on handle portion 88 thereby easily removing connector 28 from energy generator 22.

While the present invention has been illustrated by description of several embodiments, it is not the intention of the applicant to restrict or limit the spirit and scope of the appended claims to such detail. Numerous other variations, changes, and substitutions will occur to those skilled in the art without departing from the scope of the invention. For instance, the device and method of the present invention has been illustrated in relation to varicose veins, but it will be understood that the present invention has broader applicability. Additionally, positioning device 70 can alternatively include a stepper motor or ratchet mechanism attached to a holding device such as a collet or the like. Such a holding device could movably engage optical fiber 13 to position light-emitting section 19 within the treatment site L. Alternatively, wire harness 47 could be directly connected to connector 28 of energy delivery device 12 in lieu of receptacle 43 of energy generator 22. Moreover, the structure of each element associated with the present invention can be alternatively described as a means for providing the function performed by the element. It will be understood that the foregoing description is provided by way of example, and that other modifications may occur to those skilled in the art without departing from the scope and spirit of the appended Claims.

## Claims

1. A medical apparatus for the thermal treatment of human tissue comprising:
an energy delivery device including an optical fiber having a light-emitting section at a distal end thereof; and
a positioning device that engages said optical fiber for moving said light-emitting section.

2. The medical apparatus for the thermal treatment of human tissue according to claim 1, further comprising an energy generator connected to said energy delivery device, said energy generator including a main processor.

3. The medical apparatus for the thermal treatment of human tissue according to claim 2, wherein said energy generator is a source of laser light delivered through said light-emitting section of said energy delivery device.

4. The medical apparatus for the thermal treatment of human tissue according to claim 3, wherein said positioning device is operatively connected to said energy generator and is controlled using said main processor.

5. The medical apparatus for the thermal treatment of human tissue according to claim 4, wherein said optical fiber includes a temperature sensor adjacent said light-emitting section for optically measuring a temperature.

6. The medical apparatus for the thermal treatment of human tissue according to claim 5, wherein said energy delivery device includes a memory device.

7. The medical apparatus for the thermal treatment of human tissue according to claim 6, wherein said memory device has at least one parameter stored therein and wherein said main processor compares the temperature measurement to at least one of said parameters.

8. The medical apparatus for the thermal treatment of human tissue according to claim 7, wherein said main processor automatically controls the movement of said light-emitting section within a treatment site and also adjusts the energy delivered from said energy generator to said light-emitting section in response to the temperature measurement.

9. A medical apparatus for the treatment of a lumen, said medical apparatus comprising an energy delivery device including a sensor, said energy delivery device connected to an energy generator and engaging a positioning device, said energy delivery device emitting energy received from said energy generator, said positioning device automatically moving said energy delivery device in response to signals received from said sensor.

10. A medical apparatus for the thermal treatment of human tissue comprising an energy generator having a main processor and being operatively connected to an energy delivery device, said energy delivery device including an optical fiber, said optical fiber having a light-emitting section and a temperature sensor at a distal end thereof, said temperature sensor optically measures a temperature within said human tissue when said light-emitting section is energized by said energy generator, a positioning device engages said optical fiber, said positioning device controlled by said main processor and wherein said positioning device controls the movement of said light-emitting section within said human tissue.
